# EUROPEAN PATENT SPECIFICATION

(11) **EP 3 653 153 B1**
(45) Date of publication and mention of the grant of the patent: **07.10.2026**
(21) Application number: 19209784.8
(22) Date of filing: 18.11.2019
(51) Int. Cl.: A61B 18/14, A61M 25/10

(54) **MEDICAL PROBE WITH WIRING DISPOSED BETWEEN TWO EXPANDABLE MEMBRANES**
MEDIZINISCHE SONDE MIT EINER VERDRAHTUNG ZWISCHEN ZWEI EXPANDIERBAREN MEMBRANEN
SONDE MÉDICALE COMPORTANT UN CÂBLAGE DISPOSÉ ENTRE DEUX MEMBRANES EXPANSIBLES

(30) Priority: 19.11.2018 US 201862769424 P; 18.10.2019 US 201916657319
(43) Date of publication of application: 20.05.2020
(73) Proprietor: Biosense Webster (Israel) Ltd., Yokneam, 2066717 (IL)
(72) Inventor: VALLS, Jace P., Irwindale, CA 91706 (US); GALLARDO, Diana S., Irwindale, CA 91706 (US); PERKINS, Charles E., Danvers, MA 01923 (US); NGO-CHU, Don Q., Pasadena, CA 91107 (US)
(74) Representative: Carpmaels & Ransford LLP

(56) References cited:
- WO-A1-2014/168987
- US-A1- 2017 312 022
- US-A1- 2017 348 049
- US-A9- 2016 374 748

## Description

### BACKGROUND

Ablation of cardiac tissue has been used to treat cardiac arrhythmias. Ablative energies are typically provided to cardiac tissue by a tip portion which can deliver ablative energy alongside the tissue to be ablated. Some of these catheters administer ablative energy from various electrodes three-dimensional structures. Ablative procedures incorporating such catheters may be visualized using fluoroscopy.

Patent application publication US2016/0374748 describes an expandable energy delivery assembly adapted to deliver electrical energy to tissue. The assembly includes an elongate device including an irrigation shaft defining an irrigation lumen fluidly couplable to an irrigation source and a rapid exchange shaft defining a guidewire lumen configured for reception and passage of a guidewire. The assembly also includes an inflatable element that is secured to the elongate device. The inflatable element includes a double helical electrode disposed on the inflatable element that makes between about 0.5 and 1.5 revolutions around the inflatable element.

Patent application publication US2017/0348049A1 describes systems, devices and methods for modulating targeted nerve fibres (e.g., hepatic neuromodulation) or other tissue. Systems, devices and methods for cooling energy delivery members are also described. The systems may be configured to access tortuous anatomy of or adjacent hepatic vasculature. The systems may be configured to target nerves surrounding an artery or other blood vessel, such as the common hepatic artery.

Patent application publication WO2014/168987A1 describes cardiac ablation catheters and methods of use. The catheter may include at least one camera inside an expandable membrane for visualizing an ablation procedure.

Patent application publication US2017/0312022A1 describes an irrigated balloon catheter for use in an ostium of a pulmonary vein, including a flew circuit electrode assembly adapted for circumferential contact with the ostium when the balloon is inflated. Adapted for both diagnostic and therapeutic applications and procedures, the balloon catheter may be used with a lasso catheter or focal catheter.

### SUMMARY OF THE INVENTION

The invention is defined in the appended claims.

### SUMMARY OF THE DISCLOSURE

In the manufacturing of a medical probe having a balloon and electrodes arrayed on the balloon, we have determined that the electrical connection between a thermocouple (i.e., temperature sensor) on each electrode may not be optimal when the thermocouple/electrode is connected to wires that extends from inside the balloon due to the number of wires (referenced also as a "bifilar") connecting to the ten or more electrodes on the balloon. Accordingly, applicant has devised an electrophysiology catheter having a balloon with three expandable membranes attached to each other such that the wires no longer extend from inside the first membrane yet at the same time is captured between the first membrane and a second membrane.

Specifically, we have devised an electrophysiology probe that includes a tubular member, a first expandable membrane, a second expandable membrane, a plurality of electrodes with a plurality of wires connected to the electrodes. The tubular member extends along a longitudinal axis from a first end to a second end with a first expandable membrane having an outer surface and an inner surface disposed about the longitudinal axis. The first expandable membrane has a first expandable membrane portion being coupled to the second end of the tubular member and second expandable membrane portion spaced apart from the first expandable membrane portion along the longitudinal axis. The first expandable membrane is configured to be expanded from a compressed shape to a balloon shaped member. The plurality of electrodes are disposed on the outer surface of the first expandable membrane with one or more wires connected to each of the plurality of electrodes. Each wire extends from the tubular member to the electrode. The second expandable membrane encapsulates the one or more wires between the second expandable membrane and the first expandable membrane so that the wires are constrained between the first and second expandable membrane with the electrodes exposed to ambient environment.

The probe is also configured to have the following features, which can be combined into various combinations or permutations, such as, for example, the tubular member defines a first lumen that extend from the first end to the second end so that the wires are contained in the first lumen; the tubular member defines a second lumen that extends through the first expandable membrane to allow for another instrument to pass through the second lumen; the tubular member defines a third lumen that extends to a portion within the first expandable membrane, the third lumen configured to allow irrigation fluid to be delivered to the portion within the first expandable membrane; the first expandable membrane includes a plurality of openings that extends through the membrane to allow for irrigation fluid to flow from inside the membrane to outside of the membrane; the electrodes extend from a central electrode portion equiangularly about the longitudinal axis from the first expandable membrane portion towards the second expandable membrane portion such that the second expandable membrane encapsulates a portion of each of the electrodes proximate the second expandable membrane portion; wherein each of the plurality of electrodes defines a fishbone pattern not covered by the second expandable membrane; each electrode is coupled to the outer surface of the first expandable membrane via a substrate; a radiopaque marker is defined by a fish-head portion of each electrode; each electrode includes a radiopaque marker having a configuration different from other radiopaque markers on the other electrodes; a third expandable membrane disposed proximate the first expandable membrane portion so that the third expandable membrane encircles a portion of the first expandable membrane about the longitudinal axis; the third expandable membrane encapsulates a portion of a substrate for each of the plurality of electrodes between the first expandable membrane and the third expandable membrane; a retaining ring disposed about the third expandable membrane; the first expandable membrane includes a circumferential surface area of approximately 52% of a total surface area of the first expandable membrane that is not covered by the second and third expandable membranes; each substrate for each electrode includes a substrate surface area approximately 8 percent of the exposed outer circumferential surface area of the first expandable membrane; the plurality of electrodes comprises ten electrodes disposed equiangularly about the longitudinal axis on the first membrane; the second expandable membrane and third expandable membrane cover approximately half of the outer surface area of the first expandable membrane; the first expandable membrane includes a generally spheroidal member with a diameter as referenced to the longitudinal axis of about 30 millimeters and wherein the second expandable membrane and the third expandable membrane each includes a hemi-spherical member; or the first expandable membrane includes a first configuration disposed in a tubular member of less than 8 French and a second configuration disposed outside the tubular member of about 30 millimeters as referenced to the longitudinal axis.

### BRIEF DESCRIPTION OF THE DRAWINGS

While the specification concludes with the claims, which particularly point out and distinctly claim the subject matter described herein, it is believed the subject matter will be better understood from the following description of certain examples taken in conjunction with the accompanying drawings, in which like reference numerals identify the same elements and in which:
FIG. 1 is an exploded perspective view of the medical probe which shows a base balloon or first expandable membrane with radiating electrode assemblies that are partially covered by respective second and third expandable membranes;
FIG. 2 illustrates an assembled medical probe of Fig. 1;
FIG. 3A is a side view of the medical probe of Fig. 2;
FIG. 3B is a blown-up side view of a portion of the membrane of Fig. 3A;
FIG. 3C illustrates a lateral or circumferential surface area (shaded portion) not covered by the hemispherical second and third expandable membranes of Fig. 1.
FIG. 4 is a color photograph of an actual prototype according to an embodiment described and illustrated herein.
FIG. 5 is a color photograph of yet another prototype of the embodiments described and illustrated herein.

### MODES OF CARRYING OUT THE DISCLOSURE

The following detailed description should be read with reference to the drawings, in which like elements in different drawings are identically numbered. The drawings, which are not necessarily to scale, depict selected embodiments and are not intended to limit the scope of the disclosure.

The detailed description illustrates by way of example, not by way of limitation, the principles of the disclosure.

This description will clearly enable one skilled in the art to make and use the disclosure, and describes several embodiments, adaptations, variations, alternatives and uses of the disclosure, including what is presently believed to be the best mode of carrying out the disclosure.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems to human use, although use of the subject disclosure in a human patient represents a preferred embodiment. As well, the term "proximal" indicates a location closer to the operator whereas "distal" indicates a location further away to the operator or physician.

Ablation of cardiac tissue to correct a malfunctioning heart is a well-known procedure for implementing such a correction. Typically, to successfully ablate, cardiac electro potentials need to be measured at various locations of the myocardium. In addition, temperature measurements during ablation provide data enabling the efficacy of the ablation to be measured. Typically, for an ablation procedure, the electropotentials and the temperatures are measured before, during, and after the actual ablation. The medical probe used in the ablation procedure are therefore known as an electrophysiology probe. Such probe may have a handle connected to a mapping and diagnostic system, which can be understood from commonly owned US Patent Application S.N. 15/939,154 filed on March 28, 2018 (Attorney Docket No. 400528-20009 [BIO5959USNP]) entitled IRRIGATED ELECTROPHYSIOLOGY CATHETER WITH DISTINGUISHABLE ELECTRODES FOR MULTI-ELECTRODE IDENTIFICATION AND ORIENTATION UNDER 2-D VISUALIZATION.

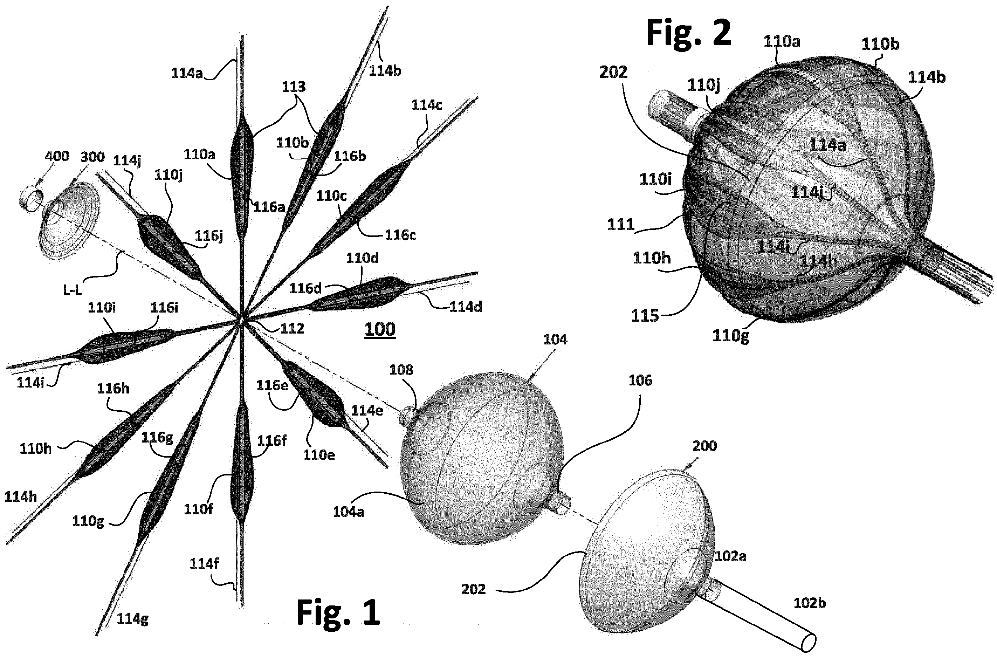

Figure 1 illustrates an exploded perspective view of an electrophysiology probe 100 that includes a tubular member 102 extending along a longitudinal axis L-L from a first (proximal) end 102b to a second (or distal) end 102a. A first expandable membrane 104 is attached to the tubular member 102 near the distal end 102b. The membrane 104 has an outer surface 104a and an inner surface 104b disposed about the longitudinal axis L-L. The outer surface 104a is exposed to the ambient environment while the inner surface 104b is exposed to the internal volume of the balloon defined by the membrane 104. The first expandable membrane 104 has a first expandable distal membrane portion 108 being coupled to the second end 102a of the tubular member 102 and second expandable distal membrane portion 106 spaced apart from the first expandable distal membrane portion 108 along the longitudinal axis L-L.

It is noted that first expandable membrane 104 is configured to be expanded from a compressed shape (generally tubular configuration) to a balloon (or generally spheroidal) shaped member. A plurality of electrodes (110a, 110b, 110c, 110d, 110e, 110f, 110g, 110h, 110i and 110j, which may be referred to singularly or collectively as "electrode 110") are disposed on the outer surface 104a of the first expandable membrane 104. The electrodes 110 are arranged so that they radiate from a generally common center or centroid substrate 112 near the second expandable distal membrane portion 108 which is distal to the tubular member 102. The electrodes 110a-110j may have one or more wires, i.e., bifilar 114a-114j, respectively, connected to each of the plurality of electrodes 110a-110j via a connection junction 116a-116j. Each of the wires 114a-114j (which may be singular in form "wire" or plural "wires" will be collectively referred to as "wire 114") is connected to the connection point at the "underside" surface of the electrode 110. The underside surface of each electrode 110 is the electrode surface that is not exposed to the ambient environment and is typically bonded to the outer surface 104a of the membrane 104. As the connection point 116 (typically a solder point) is generally at the center of the electrode, the wire is covered by the underside surface of each electrode. However, as each wire or bifilar 114a-114j extends toward the tubular member 102, the electrode surface or the substrate on which the electrode is bonded thereto becomes smaller thereby leaving the wire or bifilars 114a-114j exposed.

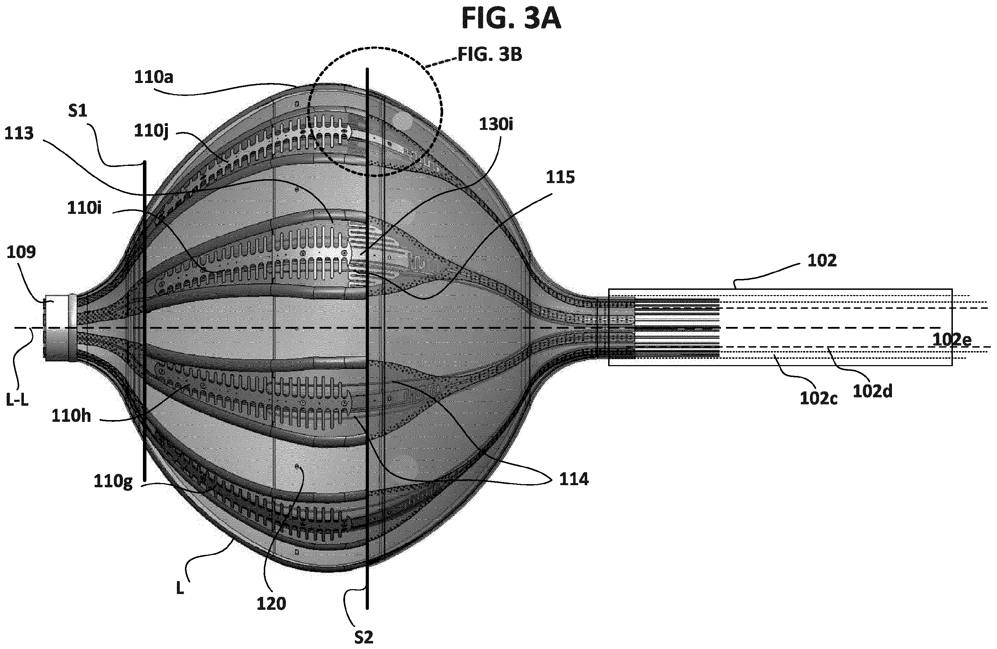

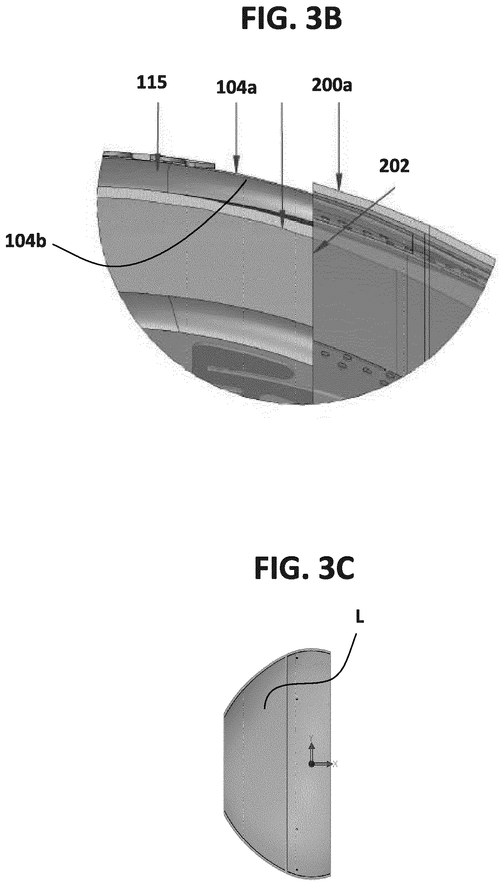

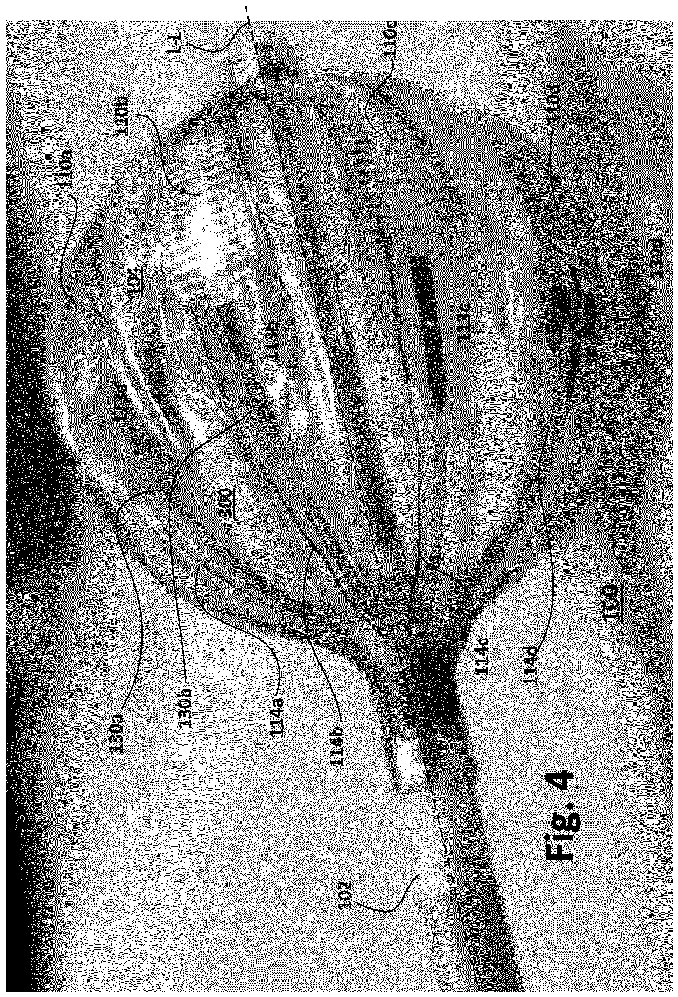

As can be seen in Fig. 2, when group of wires 114a-114j are mounted on the membrane 104, each wire 114 is configured to extend from the tubular member 102 to the respective electrode 110 such that each wire follows the topographic outer surface 104a of membrane 104. In extending the wires 114 toward the tubular member 102, the wires 114 become exposed to the ambient environment (e.g., biological tissues or blood) as each wire 114 leaves the underside surface of each electrode or the underside surface of the substrate 113 (Fig. 3A). As each wire 114 may be used to conduct or transmit electrical energy or signals, it would be detrimental to expose the wires 114 to the ambient biological tissue environment. As such, we have devised a second expandable membrane 200 that encapsulates the one or more wires (114a-114j) between the second expandable membrane 200 and the first expandable membrane 104 so that the wires 114a-114j are constrained between the first and second expandable membrane (Fig. 4). Such configuration eliminates the exposure of the wires to the ambient environment yet still allowing the electrodes/thermocouples to be exposed to biological tissues so that the electrodes and thermocouples to work for their intended purposes. Moreover, as the wires 114 are constrained or captured between the first and second membranes, there is virtually no likelihood of the wires being entangled or mis-connected to the wrong electrode or thermocouple during assembly. In the preferred embodiment, each wire of the bifilar is coupled to a temperature sensor in the form of a thermocouple 116 disposed on or near each electrode 110.

It is noted that tubular member 102 defines a first internal passageway in the form of a lumen 102c, shown here as dashed lines in Fig. 3A, that extends from the first end 102a to the second end 102b of tubular member 102 so that the one or more wires are disposed in the first lumen 102c. To allow other instruments (e.g., guide wires, optical sensor etc.,) to be delivered through the balloon 104 (and outside of the distal-most portion 109 of balloon) the tubular member 102 can be provided with a second lumen 102d that extends through the membrane portions 106 and 108 to allow for another instrument to pass through the second lumen 102d. Additionally, the tubular member 102 can be provided with yet another internal passageway in the form of a third lumen 102e. Irrigation fluid can be provided in either of the second lumen 102d or third lumen 102e so that the irrigation fluid flows into the internal volume of the membrane 104, through openings or pores 120 provided through the membrane inner surface 104b and outer surface 104a to outside of the membrane 104 to the ambient environment (e.g., biological tissues or organ). Each electrode may have four irrigation openings formed on the electrode such that the electrode irrigation openings are aligned with the pores 120 of the membrane. In the preferred embodiment, lumen 102c, lumen 102d and 102e are configured or extruded as concentric passageways, in the form of a tube 102e within tube 102d within a tube 102c with outer tubular member 102. Tubular member 102 can be a suitable biocompatible polymer as is known to those skilled in the art.

Referring to Fig. 2, the plurality of electrodes 110a-110j extend from a substrate centroid 112 equiangularly about the longitudinal axis L-L from the first expandable distal membrane portion 108 towards the second expandable distal membrane portion 106 such that the second expandable membrane 200 encapsulates a portion of each of the electrodes (110a-110j) proximate the second expandable membrane portion 106. The second expandable membrane 200 has a border 202 (Fig. 1) that extends over a proximal portion (i.e., fish-head 115) of the electrode 110 outer surface (Fig. 2) while allowing the electrode fish-bone pattern 110 to be exposed to the ambient environment.

That is, each of the plurality of electrodes 110a-110j defines a fishbone pattern not covered by the second expandable membrane 200 to allow the fishbone electrodes to be exposed to the ambient environment. Each electrode (110a-110j) is coupled to the outer surface of the first expandable membrane 104 via a substrate 113 which itself is connected to or bonded to the outer surface 104a of the first expandable membrane 104. The electrode 110a-110j can have a portion of its perimeter bonded directly to membrane 104. A suitable seal 111 can be formed so that the seal 111 runs along the outer perimeter of the substrate 113 of each electrode (110a-110j). In a preferred embodiment, the seal 111 can be provided in the form of a polyurethane seal.

Referring to Fig. 3A, a radiopaque marker 130 is defined by a proximal fish-head portion of each electrode such that there can be respective radiopaque markers 130a, 130b, 130c, 130d, 130e, 130f, 130g, 130h, 130i and 130j for corresponding electrodes 110a-110j. To ensure that the location of each electrode can be determined while inside a body organ with x-rays, each electrode 110 may have a radiopaque marker (130a-130j) with each marker having a configuration different from other radiopaque markers on the other electrodes.

Referring to Fig. 1, a third expandable membrane 300 can be disposed proximate the first expandable distal membrane portion 108 so that the third expandable membrane 300 encircles an outer surface portion of the first expandable membrane 104 about the longitudinal axis L-L proximate the distal portion 109 of the membrane 104. The third expandable membrane encapsulates a portion of the substrate 113 (Fig. 3A) for each of the plurality of electrodes near distal portion 109 of membrane 104. Preferably, the third expandable membrane 300 allows for encapsulation of the substrates 113 of each electrode (110a-110j) as the substrates 113 converge to centroid 112 near the distal portion 109 of the membrane 104. A retaining ring 109 is disposed about the third expandable membrane 300 (near distal portion 108 of membrane 104) to hold the third expandable membrane 300 as well as the substrates 113 to the first expandable membrane 104. The third expandable membrane 300 can be bonded to the first expandable membrane 104 thereby capturing the substrate 113 therebetween the two membranes (104 and 300).

Referring to Fig. 3A, it is noted that the surface area of the membrane 104 that is exposed (i.e., not covered) by second expandable membrane 200 and third expandable membrane has a circumferential surface area L delineated between a virtual slice S1 (defined by the intersection of third expandable membrane 300 with first expandable membrane 104) orthogonal to axis L-L and virtual slice S2 orthogonal to the longitudinal axis L-L whereby slice S2 is defined by the intersection of the second expandable membrane 200 to the first expandable membrane 104. For clarity, it can be seen in Fig. 3C that if the first expandable membrane 104 approximates a sphere (when membrane 104 is expanded to its service characteristic) then the circumferential surface area L can be determined once the parameters of the spheroid body is known. In the preferred embodiment, shown in Fig. 4, the first expandable membrane 104 includes a circumferential surface area L (Figs. 3A and 3C) of approximately 52% of a total surface area of the first expandable membrane 104. That is, the circumferential surface area L is the exposed surface area (without any electrode or substrate) of first expandable membrane 104 or outer circumferential area of first expandable membrane 104 that is also not covered by the second expandable membrane 200 and third expandable membrane 300. Further, it is noted that each substrate 113 for each electrode 110 includes a substrate surface area approximately 8% of the exposed outer circumferential surface area L of the first expandable membrane 104. In the preferred embodiments, the second expandable membrane 200 and third expandable membrane 300 cover approximately half of the outer surface area of the first expandable membrane 104.

In the preferred embodiments, the first expandable membrane includes a generally spheroidal member with a diameter as referenced to the longitudinal axis L-L of about 30 millimeters and the second expandable membrane and the third expandable membrane each includes a hemi-spherical member with the respective major diameter of each hemispherical member being less than 30 mm. In the preferred embodiments, the total surface area of membrane 104 is about 4500 squared-mm while the circumferential surface area L is about 2400 squared-mm and each flexible substrate 113 is about 200 squared-mm when the membrane 104 is at its fully expanded (i.e., designed) configuration, shown exemplarily in Fig. 4.

The balloon 104 of the diagnostic/therapeutic catheter has an exterior wall or membrane 104a of a bio-compatible material, for example, formed from a plastic such as polyethylene terephthalate (PET), polyurethane or PEBAX^{®}. The tubular shaft 102 and the distal shaft end 102a define a longitudinal axis L-L of the balloon 104. The balloon 104 is deployed, in a collapsed configuration as described in commonly-owned US Patent Application S.N. 15/939,154 filed on March 28, 2018 (Attorney Docket No. 400528-20009 [BIO5959USNP]) (via the lumen 23 of the probe 20 in this prior application). The membrane 104a of the balloon 104 is formed with irrigation pores or apertures 120 (shown in FIG. 3A) through which the fluid (e.g., saline) can exit from the interior of the balloon 104 to outside the balloon for cooling the tissue ablation site at the ostium.

As described earlier in relation to Fig. 2, membrane 24 supports and carries a combined electrode and temperature sensing member which is constructed as a multi-layer flexible circuit electrode assembly 110a-110j. The "flex circuit electrode assembly" 110a-110j may have many different geometric configurations than as shown here. In the illustrated embodiment, the flex circuit electrode assembly 110a-110j has a plurality of radiating substrates or strips 113a-113j, as best seen in FIG. 1. The substrates 113a-113j are evenly distributed about the distal end 109 and the balloon 104. Each substrate 113a-113j has wider proximal portion that gradually tapers to a narrower distal portion as referenced to the longitudinal axis.

For simplicity, the flex circuit electrode assembly 110 is described with respect to one of its substrate 113 as shown in FIG. 3A, although it is understood that following description may apply to each substrate 113 of the assembly 110. The flex circuit electrode assembly 110 includes a flexible and resilient sheet substrate material 113, constructed of suitable bio-compatible materials, for example, polyimide. In some embodiments, the sheet substrate material 113 has a greater heat resistance (or a higher melting temperature) compared to that of the balloon membrane 104. In some embodiments, the substrate material 113 is constructed of a thermoset material having a decomposition temperature that is higher than the melting temperature of the balloon membrane 104 by approximately 100 degrees Celsius or more.

The substrate material 113 is formed with one or more irrigation pores or apertures (not labeled) that are in alignment with the irrigation apertures 120 of the balloon member 104 so that fluid passing through the irrigation apertures 120 and (not labeled) can pass to the ablation site on the ostium.

The substrate material 113 has a first or outer surface facing away from the balloon membrane 104, and a second or inner surface facing the balloon membrane 104. On its outer surface, the substrate material 113 supports and carries the contact electrodes 110. The configuration or trace of the contact electrode 110 may resemble a "fishbone" but it should be noted that the disclosure is not limited to such configuration. In contrast to an area or "patch" ablation electrode, the fingers of the contact electrode 110 advantageously increase the circumferential or equatorial contact surface of the contact electrode 110 with the ostium while void regions between adjacent fingers advantageously allow the balloon 104 to collapse inwardly or expand radially as needed at locations along its equator. In the illustrated embodiment, the fingers have different lengths, some being longer, others being shorter. For example, the plurality of fingers includes a distal finger, a proximal finger and fingers therebetween, where each of the fingers in between has a shorter adjacent finger. For example, each finger has a length different from its distal or proximal immediately adjacent neighboring finger(s) such that the length of each finger generally follows the tapered configuration of each substrate 113. In the illustrated embodiment, there are 22 fingers extending across (past each lateral side of) the elongated portion. In some embodiments, the contact electrode 110 includes gold with a seed layer between the gold and the membrane 104. The seed layer may include titanium, tungsten, palladium, silver, or combinations thereof.

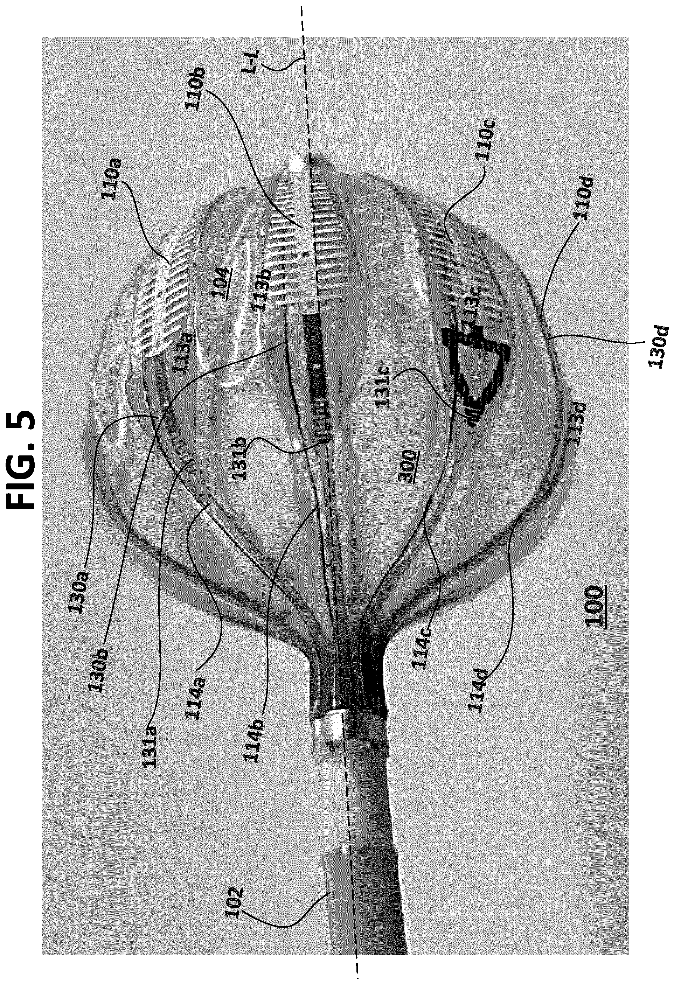

As shown in Fig. 5, the flexible electrode may have its radiopaque marker in the variation identified as 131a, 131b, 131c and so on to assist in the identification of the electrode being energized. The markers 131a-131j have various serpentine configurations (as compared to Fig. 4) to allow for increased flexibility due to the presence of the second membrane 200 which tend to reduce the flexibility of the device near the markers 131a-131j.

Description of the diagnostic/therapeutic catheter in accordance with the foregoing disclosure may be found in U.S. Patent Application No. 15/360,966, published as U.S. Patent Application Publication No. 2017/0312022.

Any of the examples or embodiments described herein may include various other features in addition to or in lieu of those described above. The teachings, expressions, embodiments, examples, etc. described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the systems described herein may be accomplished by appropriate modifications without departing from the scope of the claims. In addition, where steps described above indicate certain events occurring in certain order, it is intended that certain steps do not have to be performed in the order described but, in any order, if the steps allow the embodiments to function for their intended purposes. Therefore, to the extent there are variations of the disclosure, which are within the scope of the invention as defined by the appended claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. An electrophysiology probe (100) comprising:
a tubular member (102) extending along a longitudinal axis from a first end (102b) to a second end (102a);
a first expandable membrane (104) having an outer surface (104a) and an inner surface (104b) disposed about the longitudinal axis, the first expandable membrane having a first expandable membrane portion (108) and a second expandable membrane portion (106) spaced apart from the first expandable membrane portion along the longitudinal axis, the first expandable membrane being coupled to the second end (102a) of the tubular member (102) and wherein the first expandable member (104) is configured to be expanded from a compressed shape to a balloon shaped member;
a plurality of electrodes (110) disposed on the outer surface of the first expandable membrane (104);
one or more wires (114) connected to each of the plurality of electrodes, each wire extending from the tubular member to the electrode;
a second expandable membrane (200) that encapsulates the one or more wires between the second expandable membrane (200) and the first expandable membrane (104) so that the wires are constrained between the first expandable membrane (104) and the second expandable membrane (200) with the electrodes exposed to ambient environment;
**characterised by**
a third expandable membrane (300) disposed proximate the first expandable membrane portion (108) so that the third expandable membrane (300) encircles a distal portion of the first expandable membrane (104) about the longitudinal axis, wherein the third expandable membrane (300) encapsulates a portion of a substrate (113) for each of the plurality of electrodes between the first expandable membrane and the third expandable membrane.

2. The probe of the previous claim, wherein the tubular member defines a first lumen (102c) that extend from the first end to the second end so that the wires are contained in the first lumen.

3. The probe of one or more of the previous claims, wherein the tubular member defines a second lumen (102d) that extends through the first expandable membrane to allow for another instrument to pass through the second lumen.

4. The probe of one or more of the previous claims, wherein the tubular member defines a third lumen (102e) that extends to a portion within the first expandable membrane, the third lumen configured to allow irrigation fluid to be delivered to the portion within the first expandable membrane.

5. The probe of one or more of the previous claims, wherein the first expandable membrane (104) includes a plurality of openings (120) that extends through the membrane (104) to allow for irrigation fluid to flow from inside the membrane to outside of the membrane.

6. The probe of one or more of the previous claims, wherein the electrodes (110) extend from a central electrode portion equiangularly about the longitudinal axis from the first expandable membrane portion (108) towards the second expandable membrane portion (106) such that the second expandable membrane encapsulates a portion of each of the electrodes proximate the second expandable membrane portion.

7. The probe of one or more of the previous claims, wherein each of the plurality of electrodes (110) defines a fishbone pattern not covered by the second expandable membrane (200).

8. The probe of one or more of the previous claims, wherein the substrate (113) for each electrode is coupled to the outer surface of the first expandable membrane.

9. The probe of one or more of the previous claims, wherein a radiopaque marker (130) is defined by a fish-head portion of each electrode.

10. The probe of one or more of the previous claims, wherein each electrode (110) includes a radiopaque marker (130) having a configuration different from other radiopaque markers on the other electrodes.

11. The probe of one or more of the previous claims, further comprising a retaining ring disposed about the third expandable membrane (300).

12. The probe of one or more of the previous claims, wherein the first expandable membrane (104) includes a circumferential surface area of approximately 52% of a total surface area of the first expandable membrane (104) that is not covered by the second and third expandable membranes.

13. The probe of one or more of the previous claims, wherein each substrate (113) for each electrode (110) includes a substrate surface area approximately 8 percent of the exposed outer circumferential surface area of the first expandable membrane.

14. The probe of one or more of the previous claims, wherein the plurality of electrodes (110) comprises ten electrodes disposed equiangularly about the longitudinal axis on the first membrane.

15. The probe of one or more of the previous claims, wherein the second expandable membrane and third expandable membrane cover approximately half of the outer surface area of the first expandable membrane.

16. The probe of one or more of the previous claims, wherein the first expandable membrane includes a generally spheroidal member with a diameter as referenced to the longitudinal axis of about 30 millimeters and wherein the second expandable membrane and the third expandable membrane each includes a hemi-spherical member.

17. The probe of one or more of the previous claims, wherein the first expandable membrane includes a first configuration disposed in a tubular member of less than 8 French and a second configuration disposed outside the tubular member of about 30 millimeters as referenced to the longitudinal axis.

## Patentansprüche

1. Elektrophysiologiesonde (100), umfassend:
ein rohrförmiges Element (102), das sich entlang einer Längsachse von einem ersten Ende (102b) zu einem zweiten Ende (102a) erstreckt;
eine erste expandierbare Membran (104), die eine äußere Oberfläche (104a) und eine innere Oberfläche (104b) aufweist, die um die Längsachse herum angeordnet sind, wobei die erste expandierbare Membran einen ersten expandierbaren Membranabschnitt (108) und einen zweiten expandierbaren Membranabschnitt (106) aufweist, der von dem ersten expandierbaren Membranabschnitt entlang der Längsachse beabstandet ist, wobei die erste expandierbare Membran mit dem zweiten Ende (102a) des rohrförmigen Elements (102) gekoppelt ist und wobei das erste expandierbare Element (104) konfiguriert ist, um von einer komprimierten Form zu einem ballonförmigen Element expandiert zu werden;
eine Vielzahl von Elektroden (110), die auf der äußeren Oberfläche der ersten expandierbaren Membran (104) angeordnet sind;
einen oder mehrere Drähte (114), die mit jeder der Vielzahl von Elektroden verbunden sind, wobei sich jeder Draht von dem rohrförmigen Element zu der Elektrode erstreckt;
eine zweite expandierbare Membran (200), die den einen oder die mehreren Drähte zwischen der zweiten expandierbaren Membran (200) und der ersten expandierbaren Membran (104) einkapselt, sodass die Drähte zwischen der ersten expandierbaren Membran (104) und der zweiten expandierbaren Membran (200) eingeklemmt sind, wobei die Elektroden der Umgebung ausgesetzt sind; **gekennzeichnet durch**
eine dritte expandierbare Membran (300), die nahe dem ersten expandierbaren Membranabschnitt (108) angeordnet ist, sodass die dritte expandierbare Membran (300) einen distalen Abschnitt der ersten expandierbaren Membran (104) um die Längsachse herum umgibt, wobei die dritte expandierbare Membran (300) einen Abschnitt eines Substrats (113) für jede der Vielzahl von Elektroden zwischen der ersten expandierbaren Membran und der dritten expandierbaren Membran einkapselt.

2. Sonde des vorstehenden Anspruchs, wobei das rohrförmige Element ein erstes Lumen (102c) definiert, das sich von dem ersten Ende zu dem zweiten Ende erstreckt, sodass die Drähte in dem ersten Lumen eingeklemmt sind.

3. Sonde nach einem oder mehreren der vorstehenden Ansprüche, wobei das rohrförmige Element ein zweites Lumen (102d) definiert, das sich durch die erste expandierbare Membran erstreckt, um einem weiteren Instrument zu ermöglichen, durch das zweite Lumen durchzutreten.

4. Sonde nach einem oder mehreren der vorstehenden Ansprüche, wobei das rohrförmige Element ein drittes Lumen (102e) definiert, das sich zu einem Abschnitt innerhalb der ersten expandierbaren Membran erstreckt, wobei das dritte Lumen konfiguriert ist, um Spülflüssigkeit zu ermöglichen, an den Abschnitt innerhalb der ersten expandierbaren Membran abgegeben zu werden.

5. Sonde nach einem oder mehreren der vorstehenden Ansprüche, wobei die erste expandierbare Membran (104) eine Vielzahl von Öffnungen (120) einschließt, die sich durch die Membran (104) erstrecken, um Spülflüssigkeit zu ermöglichen, von dem Inneren der Membran zu dem Äußeren der Membran zu fließen.

6. Sonde nach einem oder mehreren der vorstehenden Ansprüche, wobei sich die Elektroden (110) derart von einem zentralen Elektrodenabschnitt gleichwinklig um die Längsachse herum von dem ersten expandierbaren Membranabschnitt (108) zu dem zweiten expandierbaren Membranabschnitt (106) hin erstrecken, dass die zweite expandierbare Membran einen Abschnitt jeder der Elektroden nahe dem zweiten expandierbaren Membranabschnitt einkapselt.

7. Sonde nach einem oder mehreren der vorstehenden Ansprüche, wobei jede der Vielzahl von Elektroden (110) ein Fischgrätenmuster definiert, das nicht durch die zweite expandierbare Membran (200) bedeckt ist.

8. Sonde nach einem oder mehreren der vorstehenden Ansprüche, wobei das Substrat (113) für jede Elektrode mit der äußeren Oberfläche der ersten expandierbaren Membran gekoppelt ist.

9. Sonde nach einem oder mehreren der vorstehenden Ansprüche, wobei ein röntgendichter Marker (130) durch einen Fischkopfabschnitt jeder Elektrode definiert ist.

10. Sonde nach einem oder mehreren der vorstehenden Ansprüche, wobei jede Elektrode (110) einen röntgendichten Marker (130) einschließt, der eine Konfiguration aufweist, die sich von anderen röntgendichten Markern auf den anderen Elektroden unterscheidet.

11. Sonde nach einem oder mehreren der vorstehenden Ansprüche, ferner umfassend einen Haltering, der um die dritte expandierbare Membran (300) herum angeordnet ist.

12. Sonde nach einem oder mehreren der vorstehenden Ansprüche, wobei die erste expandierbare Membran (104) einen umlaufenden Oberflächenbereich von etwa 52 % eines Gesamtoberflächenbereichs der ersten expandierbaren Membran (104) einschließt, die nicht durch die zweite und die dritte expandierbare Membran bedeckt ist.

13. Sonde nach einem oder mehreren der vorstehenden Ansprüche, wobei jedes Substrat (113) für jede Elektrode (110) einen Substratoberflächenbereich von etwa 8 Prozent des freiliegenden äußeren umlaufenden Oberflächenbereichs der ersten expandierbaren Membran einschließt.

14. Sonde nach einem oder mehreren der vorstehenden Ansprüche, wobei die Vielzahl von Elektroden (110) zehn Elektroden umfasst, die gleichwinklig um die Längsachse herum auf der ersten Membran angeordnet sind.

15. Sonde nach einem oder mehreren der vorstehenden Ansprüche, wobei die zweite expandierbare Membran und die dritte expandierbare Membran etwa eine Hälfte des äußeren Oberflächenbereichs der ersten expandierbaren Membran bedecken.

16. Sonde nach einem oder mehreren der vorstehenden Ansprüche, wobei die erste expandierbare Membran ein grundsätzlich kugeliges Element mit einem Durchmesser bezogen auf die Längsachse von etwa 30 Millimeter einschließt und wobei die zweite expandierbare Membran und die dritte expandierbare Membran jeweils ein halbkugelförmiges Element einschließen.

17. Sonde nach einem oder mehreren der vorstehenden Ansprüche, wobei die erste expandierbare Membran eine erste Konfiguration, die in einem rohrförmigen Element angeordnet ist, von weniger als 8 Charrière, und eine zweite Konfiguration, die an dem Äußeren des rohrförmigen Elements angeordnet ist, von etwa 30 Millimeter bezogen auf die Längsachse, einschließt.

## Revendications

1. Sonde électrophysiologique (100), comprenant :
un élément tubulaire (102) s'étendant le long d'un axe longitudinal d'une première extrémité (102b) à une seconde extrémité (102a) ;
une première membrane expansible (104) ayant une surface externe (104a) et une surface interne (104b) disposées autour de l'axe longitudinal, la première membrane expansible ayant une première partie de membrane expansible (108) et une seconde partie de membrane expansible (106) espacée de la première partie de membrane expansible le long de l'axe longitudinal, la première membrane expansible étant accouplée à la seconde extrémité (102a) de l'élément tubulaire (102) et dans laquelle le premier élément expansible (104) est conçu pour être expansé d'une forme comprimée en un élément en forme de ballonnet ;
une pluralité d'électrodes (110) disposées sur la surface externe de la première membrane expansible (104) ;
un ou plusieurs fils (114) connectés à chacune de la pluralité d'électrodes, chaque fil s'étendant de l'élément tubulaire à l'électrode ;
une deuxième membrane expansible (200) qui encapsule le ou les fils entre la deuxième membrane expansible (200) et la première membrane expansible (104) de sorte que les fils soient contraints entre la première membrane expansible (104) et la deuxième membrane expansible (200) avec les électrodes exposées à l'environnement ambiant ; **caractérisée par**
une troisième membrane expansible (300) disposée à proximité de la première partie de membrane expansible (108) de sorte que la troisième membrane expansible (300) encercle une partie distale de la première membrane expansible (104) autour de l'axe longitudinal, dans laquelle la troisième membrane expansible (300) encapsule une partie d'un substrat (113) pour chacune de la pluralité d'électrodes entre la première membrane expansible et la troisième membrane expansible.

2. Sonde selon la revendication précédente, dans laquelle l'élément tubulaire définit une première lumière (102c) qui s'étend de la première extrémité à la seconde extrémité de sorte que les fils soient contenus dans la première lumière.

3. Sonde selon une ou plusieurs des revendications précédentes, dans laquelle l'élément tubulaire définit une deuxième lumière (102d) qui s'étend à travers la première membrane expansible pour permettre à un autre instrument de passer à travers la deuxième lumière.

4. Sonde selon une ou plusieurs des revendications précédentes, dans laquelle l'élément tubulaire définit une troisième lumière (102e) qui s'étend jusqu'à une partie à l'intérieur de la première membrane expansible, la troisième lumière étant conçue pour permettre à un fluide d'irrigation d'être délivré à la partie à l'intérieur de la première membrane expansible.

5. Sonde selon une ou plusieurs des revendications précédentes, dans laquelle la première membrane expansible (104) comporte une pluralité d'ouvertures (120) qui s'étendent à travers la membrane (104) pour permettre à un fluide d'irrigation de s'écouler de l'intérieur de la membrane vers l'extérieur de la membrane.

6. Sonde selon une ou plusieurs des revendications précédentes, dans laquelle les électrodes (110) s'étendent depuis une partie d'électrode centrale de manière équiangulaire autour de l'axe longitudinal depuis la première partie de membrane expansible (108) vers la seconde partie de membrane expansible (106) de telle sorte que la deuxième membrane expansible encapsule une partie de chacune des électrodes à proximité de la seconde partie de membrane expansible.

7. Sonde selon une ou plusieurs des revendications précédentes, dans laquelle chacune de la pluralité d'électrodes (110) définit un motif en arête de poisson non recouvert par la deuxième membrane expansible (200).

8. Sonde selon une ou plusieurs des revendications précédentes, dans laquelle le substrat (113) pour chaque électrode est couplé à la surface externe de la première membrane expansible.

9. Sonde selon une ou plusieurs des revendications précédentes, dans laquelle un marqueur radio-opaque (130) est défini par une partie en tête de poisson de chaque électrode.

10. Sonde selon une ou plusieurs des revendications précédentes, dans laquelle chaque électrode (110) comporte un marqueur radio-opaque (130) ayant une configuration différente des autres marqueurs radio-opaques sur les autres électrodes.

11. Sonde selon une ou plusieurs des revendications précédentes, comprenant en outre un anneau de maintien disposé autour de la troisième membrane expansible (300).

12. Sonde selon une ou plusieurs des revendications précédentes, dans laquelle la première membrane expansible (104) comporte une superficie circonférentielle d'environ 52 % d'une superficie totale de la première membrane expansible (104) qui n'est pas recouverte par les deuxième et troisième membranes expansibles.

13. Sonde selon une ou plusieurs des revendications précédentes, dans laquelle chaque substrat (113) pour chaque électrode (110) comporte une superficie de substrat d'environ 8 pour cent de la superficie circonférentielle externe exposée de la première membrane expansible.

14. Sonde selon une ou plusieurs des revendications précédentes, dans laquelle la pluralité d'électrodes (110) comprend dix électrodes disposées de manière équiangulaire autour de l'axe longitudinal sur la première membrane.

15. Sonde selon une ou plusieurs des revendications précédentes, dans laquelle la deuxième membrane expansible et la troisième membrane expansible recouvrent environ la moitié de la superficie externe de la première membrane expansible.

16. Sonde selon une ou plusieurs des revendications précédentes, dans laquelle la première membrane expansible comprend un élément généralement sphéroïdal avec un diamètre, par rapport à l'axe longitudinal, d'environ 30 millimètres et dans laquelle la deuxième membrane expansible et la troisième membrane expansible comportent chacune un élément hémisphérique.

17. Sonde selon une ou plusieurs des revendications précédentes, dans laquelle la première membrane expansible comprend une première configuration disposée dans un élément tubulaire de moins de 8 charrière et une seconde configuration disposée à l'extérieur de l'élément tubulaire d'environ 30 millimètres par rapport à l'axe longitudinal.
